# EUROPEAN PATENT SPECIFICATION

(11) **EP 1 505 964 B1**
(45) Date of publication and mention of the grant of the patent: **21.11.2007**
(21) Application number: 03752754.6
(22) Date of filing: 16.05.2003
(51) Int. Cl.: A61K 31/403, A61P 1/04

(54) **ANNELATED PYRROLE COMPOUNDS AS PROTON PUMP INHIBITORS FOR TREATING ULCER**
ANNELIERTE PYRROL-VERBINDUNGEN ALS PROTONENPUMPENHEMMER ZUR BEHANDLUNG VON GESCHWÜREN
COMPOSES DE PYRROLE ANNELES EN TANT QU'INHIBITEURS DE LA POMPE A PROTONS POUR TRAITER L'ULCERE

(30) Priority: 17.05.2002 EP 02011081; 17.05.2002 US 380928 P
(43) Date of publication of application: 16.02.2005
(73) Proprietor: MERCKLE GMBH, 89079 Ulm (DE)
(72) Inventor: SMOLKA, Adams, J., Charleston, SC 29425 (US); HAMMOND, Charles, E., Charleston, SC 29425 (US); GUPTA, Sandeep, New York, NY 10022 (US)
(74) Representative: Reitstötter - Kinzebach
(86) International application number: PCT/EP2003/005171
(87) International publication number: WO 2003/097041

(56) References cited:
- WO-A-95/32970
- WO-A-96/41626
- US-A- 5 852 033
- WALLACE, J. L. ET AL: "ML 3000 reduces gastric prostaglandin synthesis without causing mucosal injury" EUROPEAN JOURNAL OF PHARMACOLOGY (1994), 271(2/3), 525-31, XP008008664
- LAUFER S ET AL: "Gastrointestinal tolerance of [2,2-dimethyl-6-(4-chlorophenyl-7-phenyl- 2,3-dihydro-1H-pyrrolizine-5-yl]-acetic acid in the rat." ARZNEIMITTEL-FORSCHUNG, (1994 DEC) 44 (12) 1329-33., XP001108855
- TRIES, S. ET AL: "The pharmacological profile of ML3000: a new pyrrolizine derivative inhibiting the enzymes cyclo-oxygenase and 5-lipoxygenase" INFLAMMOPHARMACOLOGY (2001), 9(1-2), 113-124, XP008008717

## Description

The present invention relates to the use of the annellated pyrrole compound ML3000, a salt or ester thereof as proton pump inhibitor.

### BACKGROUND OF THE INVENTION

Peptic ulcers are one of the most prevalent diseases in industrialized nations.

Acid secretory inhibitors are among the most highly-prescribed medications, reflecting the physiological adage "No acid, no ulcer". Pharmacological or pathophysiological breaching of the gastric mucosal barriers to back diffusion of HCl leads to rapid erosion of the gastric epithelial monolayer and consequent ulceration of the mucosa. Inhibition of add secretion by antagonism at the parietal cell histamine H₂ receptor (cimetidine), or by direct covalent derivatization and inactivation of the gastric proton pump (omeprazole, lansoprazole, rabeprazole) is routine for amelioration and promotion of healing of gastric ulcers. The gastric proton pump is an enzyme which is also known as H⁺K⁺-ATPase. It is located in the membrane of gastric parietal cells and is responsible for the transport of protons from blood to lumen, which in turn results in decreasing the pH of stomach contents.

Omeprazole itself is in fact a prodrug which under acidic conditions converts to the active drug, namely its corresponding sulfenamlde. The mechanism of action of omeprazole is well-studied and is known to involve a nucleophilic attack of one (or two) thiol group(s) of the H⁺/K⁺-ATPase on the sulfur atom of the chemically active sulfenamide. The resulting chemical modification of the thiol group(s) of the enzyme (formation of a disulfide bond between the H⁺/K⁺-ATPase sulfur and the sulfur of the benzimidazole pyridinium salt) causes the observed inhibition of the proton pump. It should be emphasized however, that the conversion of the prodrug to the active enzyme inhibitor can only be achieved in acidic media which also results in substantial degradation of the active sulfenamide. In summary, the instability of omeprazole in acidic environments, which is a prerequisite to its activation into a proton pump inhibitor, is the major shortcoming of this drug.

In addition to acid secretion interieukin-8 secretion represents a further gastric mucosal function which plays an important role in gastric ulceration.

The ulcerogenic bacterium Helicobacter pylori has been shown to increase the rate and amplitude of IL-8 secretion both in vitro and in vivo, and to up-regulate IL-8 gene expression. Similar IL-8 prosecretory effects are seen as a result of IL-1b stimulation of gastric epithelial cells.

Nonsteroidal antiphlogistika (NSAIDs), such as acetylsalicylic acid (ASA), diclofenac, indomethacin, ibuprofen and naproxen, are widely used in the clinic. From a pharmacological point of view they act as inhibitors of the cyclooxygenase (COX).

The anti-inflammatory properties of NSAIDs are related to their suppression of prostaglandin synthesis. However, suppression of gastric prostaglandins decreases gastric mucosal blood flow, with concomitant mucosal sensitivity to topical injury by a variety of irritants. Gastric ulceration induced by NSAIDs significantly limits the utility of these drugs.

Pyrrolizines which pharmacologically act similar, are known from numerous publications. For instance, antiphlogistically active pyrrolizines are described in Arch. Pharm. 319, 65-69 (1986); 319, 231-234 (1986); 318, 661-663 (1985); 318, 663-664 (1985); 319, 500-505 (1988); 319, 749-755 (1986); 327, 509-514 (1994); 330, 307-312 (1997) as well as in J. Med. Chem. 1987, 30, 820-823 and 1994, 37, 1894-1897.

Further pyrrolizines can be taken from US 5,260,451 (corresponding to EP 0397175) as well as from WO 95/32970; WO 95/32971; and WO 95/32972. These compounds are represented by the structural formula 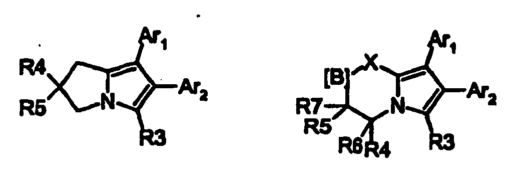 and share an annellated diarylpyrrol moiety as well as a third acidic residue R3. The compounds are characterized by a high lipophilicity, good bioavailability and half-lifes in the medium range, s. Drugs of the Future, 1995, 20 (10):1007-1009.

Further pyrrolizines of similar constitution are described in DE 198 45 446.6 and WO 01/05792. Moreover, alkylsulfinylbenzoyl and alkylsulfonylbenzoyl substituted pyrrolizines, according to US 4,232,038, are said to have anti-inflammatory, analgetic and antipyretic properties. According to DE 196 24 290.8 and DE 196 24 289.4 certain compounds of this type have a lipid-reducing action.

ML-3000 ([2,2-dimethyl-6-(4-chlorophanyl)-7-phenyl-2,3-dihydro-1 H-pyrrolizine-5 yl]-acetic acid) of the Formula (Ia)

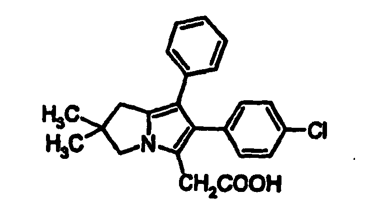

Is a non-antioxidant balanced dual inhibitor of COX and 5-Lipoxygenases (5-LO) (1). The drug is a nonselective inhibitor of COX, inhibiting both COX-1 and COX-2. This drug has analgesic, antipyretic and anti-inflammatory activity, and has been demonstrated to have potent anti-inflammatory action in a number of animal models including catrageenan-induced paw edema in the rat, and rat adjuvant arthritis (2). WO 96/41626 describes combinations of COX-2 inhibitors with 5-LO inhibitors (such as ML3000) for treatment of inflammation and inflammation related disorders. Further, it has been reported that ML3000 shows excellent gastrointestinal tolerance (12, 13). Gastroprotective properties, however, have not been observed (11).

Surprisingly, it has been found that ML-3000 has a significant gastroprotective effect. This phenomenon is associated not only with a significant inhibition of the gastric proton pump, but also with the inhibition of IL-8 secretion in gastric epithelial cells.

### SUMMARY OF THE INVENTION

Thus, the present invention relates to the use of [6-(4-chlorophenyl)-2,2-dimethyl-7-phenyl-2,3-dihydro-1H-pyrrolizine-5-yl]-acetic acid (ML3000) represented by formula (Ia): 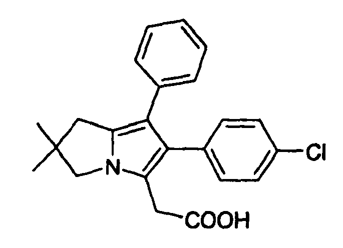 a physiologically acceptable salt or a physiologically hydrolysable ester thereof for preparing a pharmaceutical composition for treating a gastric mucosal lesion;
treating or preventing erosive gastritis;
treating or preventing non-erosive gastritis;
treating or preventing gastric ulceration; or
treating or preventing Ulcus duodeni or Ulcus ventriculi.

The present invention also relates to a combination of (i) [6-(4-chlorophenyl)-2,2-dimethyl-7-phenyl-2,3-dihydro-1H-pyrrolizine-5-yl]-acetic acid of the formula (Ia) 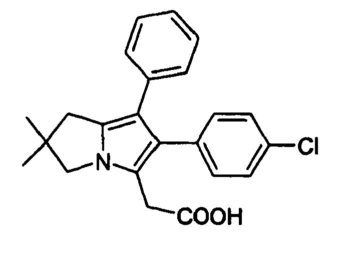 a physiologically acceptable salt or a physiologically hydrolysable ester thereof with (ii) one or more than one ulcerogenic anti-inflammatory agent for use in therapy, wherein the ulcerogenic anti-inflammatory agent is selected from the group consisting of acetylsalicylic acid (ASA), sodium salicylate, acetaminophen, phenacetin, ibuprofen, ketoprofen, indomethacin, flurbiprofen, diclofenac, naproxen, piroxicam, tebufelone, nabumetone, tenidap, alcofenac, antipyrine, amimopyrine, dipyrone, animopyrone, phenylbutazone, clofezone, oxyphenbutazone, prexazone, apazone, benzydamine, bucolome, cinchopen, clonixin, ditrazol, epirizole, fenoprofen, floctafenin, flufenamic acid, glaphenine, indoprofen, meclofenamic acid, mefenamic acid, niflumic acid, salidifamides, sulindac, suprofen, tolmetin, nabumetone, tiaramide, proquazone, bufexamac, flumizole, tinoridine, timegadine, dapsone, diflunisal, benorylate, fosfosal, fenclofenac, fentiazac, tilomisole, carprofen, fenbufen, oxaprozin, tiaprofenic acid, pirprofen, feprazone, piroxicam, sudoxicam, isoxicam, tenoxicam,
or a pharmaceutical composition comprising (i), (ii), and (iii) a pharmaceutically acceptable carrier.

Physiologically acceptable salts include acid or base addition salts.
Acid addition salts are salts of compounds of Formula (Ia) with inorganic acids, such as hydrochloric acid, sulfuric acid, nitric acid or phosphoric acid, or with organic acids, in particular carboxylic acids, e.g. acetic acid, tartaric acid, lactic acid, citric acid, malic acid, amygdalic acid, ascorbic acid, maleic acid, fumaric acid, gluconic add or sulfonic add, e.g. methanosulfonic acid, phenylsulfonic add and toluenesulfonic acid.

Base addition salts are salts of compounds of Formula (Ia) with inorganic bases, such as sodium or potassium hydroxide or with organic bases, such as mono-, di- or triethanolamine.

Physiologically hydrolyzable esters of the compounds of formula (Ia) include in particular prodrugs of the compounds of formula (Ia) which are reconverted in vivo to the compounds of formula (Ia) or an active form thereof (metabolite). Examples are alkyl (comprising the functionality COOAlkyl), aralkyl (comprising the functionality COOAlkaryl, e.g., COOAlkPhenyl), pivaloyloxymethyl, acetoxymethyl, phthalidyl, indanyl and methoxymethyl esters of the compounds of formula (Ia).

The term "alkyl" includes linear or branched alkyl groups, such as CH₃, C₂H₅, n-propyl, CH(CH₃)₂, n-butyl, CH(CH₃)-C₂H₅, isobutyl, C(CH₃)₃, n-pentyl or n-hexyl, in particular CH₃, C₂H₅ or CH(CH₃)₂, preferably having - unless otherwise stated - 1 to 8, in particular 1 to 6 and more preferably 1 to 4 carbon atoms.

-COOAlkyl means alkoxycarbonyl, such as CO-OCH₃, CO-OC₂H₅, CO-OCH₂-C₂H₅, CO-OCH(CH₃)₂, n-butoxycarbonyl, CO-OCH(CH₃)-C₂H₅, CO-OCH₂-CH(CH₃)₂, CO-OC(CH₃)₃, in particular CO-OCH₃, CO-OC₂H₅, CO-OCH(CH₃)₂ or CO-OCH₂-CH(CH₃)₂.

-COOAlkPhenyl means an alkoxycarbonyl group which is substituted on the alkyl moiety with phenyl, such as benzyloxycarbonyl.

"Aryl" preferably means naphthyl and in particular phenyl.

The term "proton pump inhibitor" as used herein will be understood to refer to those compounds which inhibit the gastric H⁺,K⁺-ATPase activity. This activity can be determined in well-known assays, e.g. in terms of inorganic phosphate release from the substrate ATP (Hongo T. et al., J.J. Pharmacol. 52: 295 (1990)). Preferred are those compounds of Formula (Ia) having a half-maximal inhibitory concentration (IC₅₀) of 50 µM or less. Using compounds of Formula (Ia) having an IC₅₀ of 5 µM or less may be of advantage according to the invention. Very effective proton pump inhibitors of Formula (Ia) have an IC₅₀ of 0,5 µM or less. According to a further aspect, those compounds of Formula (Ia) are preferred which reversibly inhibit the proton pump. According to still a further aspect, those compounds of Formula (Ia) are preferred whose proton pump-inhibiting activity is essentially pH-independent over a pH range of about 3 to 8.

Such compounds can be identified among the compounds of Formula (Ia) using well-known screening procedures such as high-throughput screening (HTS) procedures. A typical procedure comprises testing the gastric H⁺,K⁺-ATPase inhibition by each of a number of candidate compounds of Formula (1a) and identifying those which have the desired activity. It will also be appreciated that such proton pump inhibiting compounds may also possess anti-inflammatory action.

Thus, according to a particular aspect, the present invention relates to the use of proton pump inhibitors which are selected among the compounds of Formula (Ia). These compounds of Formula (Ia) are effective as gastric acid secretion inhibitors.

The compounds can be used for prevention and treatment of gastric-acid related conditions in mammals and especially in man, selected from gastritis, duodenitis, gastric ulceration, especially gastric ulcer and duodenal ulcer. Gastritis may be erosive or non-erosive, acute or chronic. In particular, the compounds are useful as antiulcer agent.

According to a particular aspect, the invention relates to the use of gastroprotective compounds of Formula (Ia). The term "gastroprotective" as used herein will be understood to refer to those compounds which are in particular capable of reducing the sensitivity of the gastrointestinal mucosa and in particular the gastric mucosa to topical injury. Such topical injury may be caused by irritants. According to a particular aspect of the invention said irritants are selected from the group consisiting essentially of NSAIDs and COX-inhibitors.

The earliest gross pathologic finding in ulcerogenesis is the erosion of the gastric epithelial monolayer. With progression of said erosion the integrity of the gastric mucosa thins, and segments of the gastrointestinal mucosa penetrate through the muscularis mucosa.

Changes in the normal mucosa defense and repair by certain factors such as NSAIDs and H. pylori, also play a role in the pathology of peptic ulcer disease.

According to one aspect of the present invention, the use of compounds of Formula (Ia) is directed to treating or preventing acid-mediated mucosal damage and subsequent ulceration. In particular, said use is directed to treating or preventing the pathologic changes involved therewith.

The present invention provides uses and pharmaceutical compositions useful therein as well as suitable packaging therefor, which are applicable to mammals which suffer from a condition as defined above or in the future may suffer from erosive gastritis, non-erosive gastritis, gastric ulceration, Ulcus duodeni or Ulcus ventriculi.

Using the compounds of Formula (1a) has particular advantages over other NSAIDs, especially those more established in use, which may actually exacerbate the progress of mucosal lesions and in particular gastric ulceration, especially when long-term application is indicated. The compounds of Formula (1a) are useful in treating such mucosal lesions and in particular gastric ulceration.

The ability of the compounds of formula (Ia) to prevent and to reverse the disease process which ultimately leads to mucosal destruction has far-reaching implications for the safe and effective treatment of mammals, especially those which need long-term anti-inflammatory therapy.

As used herein, the term "mammal(s)" denotes any mammal, preferably humans, cat, dog or horse, of which there are a large number of different breeds.

In accordance with the present invention, a treatment or prevention especially comprises ameliorating, diminishing, actively treating, reversing or preventing a condition as defined above. The expression "treating or preventing" as used herein with reference to the administration of the gastroprotective compounds of the present invention, is intended to refer to both the therapeutic objective of said administration as well as the therapeutic results actually achieved by said administration. As above-discussed, the extent of therapy accomplished by administration of said compounds may range from an amelioration to a significant diminishing of the course of the disease, and beyond to active treatment of the disease, including a reversal of the disease process.

Treating or preventing said conditions may also comprise administering in addition to one or more than one compound of Formula (Ia), one or more members selected from the group consisting essentially of antibacterially active agents, further gastroprotective agents such as further proton inhibitors and H₂-receptor antagonists, antacid agents, alginates and prokinetic agents.

Combinations with antibacterially active agents are especially useful in treating H. pylori-positive individuals (anti-H. pylori therapy). Antibacterially active agents such as clarithromycin plus metronidazole or amoxicillin in combination with proton pump inhibitors yield high eradication rates in respect of H. pylori infections. Further suitable antibacterially active agents which may be mentioned are β-lactam antibiotics, for example penicillins (such as benzylpenicillin, phenoxymethylpenicillin, propicillin, azidicillin, dicloxacillin, flucloxacillin, oxacillin, amoxicillin, bacampicillin, ampicillin, meziocillin, piperacillin or aziocillin), cephalosporins (such as cefadroxil, cefaclor, cefalexin, cefalexim, cefuroxim, cefetamet, cefadroxil, ceftibuten, cefpodoxim, cefotetan, cefazolin, cefoperazon, ceftizoxim, ceftaxim, ceftazidim, cefamandol, cefepim, cefoxitin, cefodizim, cefsulodin, ceftriaxon, cefotiam or cefmenoxim) or other β-lactam antibiotics (e.g. aztreonam, loracarbef or meropenem); enzyme inhibitors for example sulbactam, tetracyclines, for example tetracycline, oxytetracycline, minocycline or doxycycline aminoglycosides, for example tobramycin, gentamicin, neomycin, streptomycin, amikacin, netilmicin, paromomycin or spectinomycin; amphericols, for example chloramphenicol or thiamphenicol; lincomycins and macrolide antibiotics, for example clindamycin, lincomycin, erythromycin, clarithromycin, spiramycin, roxithromycin or azithromycin; polypeptide antibiotics, for example colistin, polymixin B, teloplanin or vancomycin, gyrase inhibitors, for example norfloxacin, cinoxacin, ciprofloxacin, pipemidic add, enoxacin, nalidixie add, pefloxacin, fleroxacin or ofloxacin; nitroimidazoles, for example metronidazole; or other antibiotics, for example fosfomycin or fucidic acid, where these antibacterially active substances are administered on their own or alternatively can be combined with one another.

The following compounds may be primarily mentioned as further proton pump inhibitors: omeprazole, lansoprazole, rabeprazole, leminoprazole, nepaprazole and pantoprazole.

For use in the present invention the compounds of Formula (Ia) may also be combined with other therapeutically active ingredients which would be readily apparent to the skilled artisan in this field, and which will usually be determined by the circumstances under which the therapeutic agent of the present invention is to be administered. Examples of such other therapeutically active ingredients include the above agents.

Further examples of such other therapeutically active ingredients include anti-inflammatory agents, in particular NSAIDs and additional classes of anti-inflammatory agents and examples thereof include, *e.g.*, H₁-receptor antagonists; kinin-B₁- and B₂-receptor antagonists; prostaglandin inhibitors such as PGD-, PGF- PGl₂ -, and PGE-receptor antagonists; thromboxane A₂ (TXA2-) inhibitors; PAF-receptor antagonists; gold in the form of an aurothio group together with various hydrophilic groups; immunosuppressive agents, *e.g.*, cyclosporine, azathioprine, and methotrexate; anti-inflammatory glucocorticoids, *e.g*., dexamethasone; broad-spectrum antiparasitic antibiotics, *e.g.*, the avermectins and the milbemycins; penicillamine; hydroxychloroquine; anti-gout agents, *e.g*., colchicine, xanthine oxidase inhibitors, *e.g.*, allopurinol, and uricosuric agents, *e.g.*, probenecid, sulfinpyrazone, and benzbromarone.

According to a particular aspect of the instant invention, one or more than one compound of Formula (1a) is combined with an ulcerogenic anti-inflammatory agent. Anti-inflammatory agents are ulcerogenic if they inhibit the cyclooxygenase, in particular the cyclooxygenase 1, and as a consequence, the production of certain prostaglandins, in particular prostaglandin E₂. The ulcerogenic anti-inflammatory agents are acetylsalicylic acid (ASA), sodium salicylate, acetaminophen, phenacetin, ibuprofen, ketoprofen, indomethacin, flurbiprofen, diclofenac, naproxen, piroxicam, tebufelone, etodolac, nabumetone, tenidap, alcofenac, antipyrine, amimopyrine, dipyrone, animopyrone, phenylbutazone, clofezone, oxyphenbutazone, prexazone, apazone, benzydamine, bucolome, cinchopen, clonixin, ditrazol, epirizole, fenoprofen, floctafenin, flufenamic acid, glaphenine, indoprofen, meclofenamic acid, mefenamic acid, niflumic acid, salidifamides, sulindac, suprofen, tolmetin, nabumetone, tiaramide, proquazone, bufexamac, flumizole, tinoridine, timegadine, dapsone, diflunisal, benorylate, fosfosal, fenclofenac, etodolac, fentiazac, tilomisole, carprofen, fenbufen, oxaprozin, tiaprofenic acid, pirprofen, feprazone, piroxicam, sudoxicam, isoxicam, tenoxicam. According to a particular embodiment, acetylsalicylic acid is combined with one or more than one compound of Formula (Ia).

Accordingly, this type of medication provides a means for effectively treating inflammatory conditions while having gastric sparing properties. This is particularly advantegous in situations where the administration of the ulcerogenic anti-Inflammatory agent involves the gastrointestinal tract, e.g. in case of oral administration or gastrointestinal inflammatory conditions.

In accordance with a regimen which would be used according to the invention, it is contemplated that the compounds of Formula (Ia) would be administered in combination with other medications used on a regularly scheduled basis. It is also envisioned that administration in combinations could assume a number of different forms and still be within the scope of the present invention. For example, the compounds of Formula (Ia) might simply be formulated with one or more of the other therapeutic agents which are to form the intended combination, into a convenient dosage form, such as an oral tablet, containing all of the drugs forming the combination. Varying half-lives for the different drugs could be accommodated by the person skilled in preparing formulations by creating controlled-release forms of said drugs with different release times so that relatively uniform dosing was achieved. A medicated feed used as the dosage form could also be prepared in accordance with well known principles in the art of formulation, in which the drugs used in the combination were simply present together in admixture in the feed composition. The present invention also contemplates co-administration in which the combination of drugs is achieved by the simultaneous administration of the drugs to be given in combination. Such co-administration could even be by means of different dosage forms and routes of administration. The present invention further contemplates the use of such combinations in accordance with different but regular and continuous dosing schedules whereby desired plasma levels of the drugs involved were maintained in the mammal being treated, even though the individual drugs making up the combination were not being administered to said mammal simultaneously. All such combinations would be well within the skill of the art to devise and administer.

When the compounds of formula (1a) are to be used as active ingredients according to the present invention, they can be incorporated into standard pharmaceutical dosage forms. Useful pharmaceutical compositions comprise a pharmaceutically acceptable carrier and an amount therapeutically effective for inhibiting the proton pump, of a compound of Formula (Ia) as above-defined. For example, they are useful when administered in systemic or local, oral or parenteral applications and for this purpose are combined with the usual pharmaceutical excipients, diluents and adjuvants, *e.g.*, organic and inorganic inert carrier materials such as water, gelatin, lactose, starch, magnesium stearate, talc, vegetable oils, gums, polyalkyloneglycols, etc. These pharmaceutical preparations can be employed in a solid form, *e.g.,* as tablets, capsules, and especially in combination with or for admixture with a palatable food item suitable for mammals; or they can be administered in liquid form, *e.g.*, as solutions and elixirs. Pharmaceutical excipients and adjuvants which can be added include preservatives, antioxidants, antimicrobial agents and other stabilizers; wetting, emulsifying, and suspending agents, and anticaking compounds; fragrance and coloring additives; compositions for improving compressibility, or to create a delayed-, sustained-, or controlled-release of the active ingredient; and various salts to change the osmotic pressure of the pharmaceutical preparation or to act as buffers. Particular dosage forms which have been used with success include a 5% mixed-micelle solution of ML3000 for intravenous injection, a 3% palatable paste, and oral tablets.

The therapeutically effective amount of a compound of Formula (1a) as defined may be administered systemically to said mammal, wherein said systemic administration comprises: (1) injection or infusion into suitable body tissues or cavities of a pharmaceutical composition containing said compound in suitable liquid form such as aqueous solutions, emulsions or suspensions for intraarterial, intra- or transdermal (including subcutaneous), or intraspinal especially intrathecal and most commonly intramuscular or intravenous delivery thereof; or for serving as a depot for delivery thereof; (2) instillation into suitable body tissues or cavities of a pharmaceutical composition containing said compound in suitable solid form, e.g., comprising a matrix of bio-compatible and bio-erodible materials in which particles of a solid gastroprotective compound of Formula (Ia) are dispersed, or in which, possibly, globules or isolated cells of a liquid gastroprotective compound of Formula (Ia) are entrapped, for serving as a solid implant composition for delayed-, sustained-, and/or controlled-release delivery thereof ; or (3) ingestion or administration of a pharmaceutical composition containing said compound in suitable solid or liquid form for transdermal delivery thereof, for instance a transdermal patch or a subepidermal (subcuticular) implant, for peroral delivery thereof.

A substantial number of the dosage forms described herein may be formulated so as to provide controlled-, sustained-, and/or delayed release of the active ingredient from said dosage form.

A useful controlled release dosage form of ML3000 is one which maintains a ML3000 plasma level greater than 100 ng/mL for most of the day after a single oral dose at 5 mg/kg. Preferred oral controlled release dosage forms of ML3000 are ones which maintain a plasma ML3000 concentration greater than 100 ng/mL for a period of time greater than that for which an immediate release dosage form of ML3000 maintains a comparable plasma level, when said immediate release dosage form and controlled release dosage form are administered at the same dose.

Immediate release ML3000 dosage forms containing doses of 2.5 and 5 mg/kg maintain a plasma ML3000 concentration above 100 and 200 ng/mL for 8 hours, respectively.

Preferred peroral dosage forms for systemic administration are solids, *e.g.*, palatable oral compositions such as fast dissolving palatable wafers, tablets, capsules, caplets, *etc.*, and liquids, *e.g.*, solutions, suspensions, emulsions, etc. Pharmaceutical compositions of special types suitable for oral administration to mammals may be used, and include such items as an oral paste to be delivered to the back of the tongue of the mammal being treated, a granular form to be delivered through incorporation in the mammal's food, and a chewable form wherein the active ingredient is consumed along with the palatable chew, or a chewable form which may deliver the active ingredient by leaching from the body of the chew which is not consumed, during mastication by the mammal being treated.

Said therapeutically effective amount of a compound of Formula (Ia) as defined may also be administered locally to said mammal, wherein said local administration comprises: (1) injection or infusion into a local site of gastric acid-related condition of a pharmaceutical composition containing said compound of formula (1a) in suitable liquid form for delivery thereof, including components which provide delayed-release, controlled-release, and/or sustained-release of said compound into said local site; or for serving as a depot for delivery thereof wherein said composition provides storage of said compound and thereafter delayed-, sustained-, and/or controlled-release thereof; or (2) instillation of a pharmaceutical composition containing said compound in suitable solid form for serving as a solid implant for delivery thereof, said composition optionally providing delayed-, sustained-, and/or controlled-release of said compound to said local site.

Injections may also be made of pharmaceutical compositions containing the gastroprotective compound of Formula (Ia), where the pharmaceutical composition is in delayed-release, controlled-release, or sustained-release form. These formulations of recognized composition may be a solids, semi-solids, gels or other liquid/solid combinations in which an erodible matrix or series of coatings is used to provide a continuous release of the compound of Formula (Ia) at a predetermined rate or at variable rates if desired. The terms "extended-release" and "long-acting" as well as others are used to describe these formulations. All of these employ various combinations of bioerodible polymers, *e.g.*, various cellulosic polymers, and natural materials, *e.g.*, corn starch and magnesium stearate, to obtain slow and/or uniform dispensing of the compound of Formula (Ia) contained within the matrix.

The therapeutically effective amount for treating or preventing gastric acid-related diseases, of the compound of Formula (Ia), is administered to a mammal being treated in an amount expressed as milligrams per kilogram of body weight of said mammal, per day: "mg/kg/day". The expression "per day" as used herein should not be interpreted as necessarily requiring that any particular dosage form be administered on a daily basis to the mammal being treated. The expression "per day" is merely an indication of the smallest convenient but arbitrary segment of time which is being used as part of the overall unit for measuring the dose of gastroprotective compound being administered. The dose, *i.e.,* the therapeutically effective amount of a compound of Formula (Ia) for treating or preventing gastric acid-related diseases will usually range from 0.1 mg/kg/day to 20.0 mg/kg/day, preferably from 0.1 mg/kg/day to 12.0 mg/kg/day, more preferably from 0.5 mg/kg/day to 10.0 mg/kg/day, and most preferably from 0.5 mg/kg/day to 8.0 mg/kg/day. Typical dosage forms and amounts for ML3000 would include oral administration of ML3000 at a dose rate of 2.5-5.0 mg/kg/day of body weight. Special requirements may be needed for patients having Zollinger-Ellison syndrome, such as a need for higher doses than the average patient.

It is necessary for the skilled artisan, not only to determine the preferred route of administration and the corresponding dosage form and amount, but said artisan must also determine the dosing regimen. *i.e.,* the frequency of dosing. In general terms it is most likely that the choice will be between once-a-day (*s.i.d.*) dosing and twice-a-day (*b.i.d.*) dosing, and that the former will provide more rapid and profound therapy, while the latter will provide less profound but more sustained therapy. However, this generalization does not take into account such important variables as the specific type of gastric acid-related disease involved, the specific therapeutic agent involved and its pharmacokinetics, and the specific patient (mammal) involved. For an approved product in the marketplace, much of this information is already provided by the results of clinical studies carried out to obtain such approval. in other cases, such information may be obtained in a straightforward manner in accordance with the teachings and guidelines contained in the instant specification taken in light of the knowledge and skill of the artisan. The results which are obtained can also be correlated with data from corresponding evaluations of an approved product in the same assays.

The method of the present invention can be further defined to comprises two basic steps: (I) establishing the status of a candidate mammal as presently or prospectively being in a condition of gastric add-related disease, thereby confirming that said mammal is in need of such treatment; and thereupon (II) treating or preventing said condition by administering to said mammal an amount therapeutically effective for treating or preventing said gastric acid-related disease, of a gastroprotective compound of Formula (Ia). The various aspects of Step (II) have already been discussed above in detail. Accordingly, the aspects of Step (I) will now be discussed in detail.

As far as diagnosis is concerned, it is expedient to establish the status of a mammal which is a candidate for treatment in accordance with the present invention as to whether or not the mammal is presently or prospectively in a condition of gastric acid-related disease. The expression "presently or prospectively" as used herein is intended to mean that in accordance with the below-discussed methods of making that determination, it is possible to identify a candidate mammal as either being presently in need of such treatment, or as very likely or expected to be in need of such treatment in the short term future. Prospective need of treatment may be established by those determinations of positive factors which from the experience of the artisan lead directly to the condition of gastric acid-related disease. For example, the artisan may establish from clinical examination of a mammal that it has a gastric acid-related disease, and may confirm this conclusion with further evidence from which it may be determined in accordance with established methods of measurement that the mammal will develop a gastric acid-related disease within the short term future.

The status of said mammal as presently or prospectively being in said condition of gastric acid-related disease, and thus in need of such treatment, is in particular determined by positive results from the clinical examination and evaluation of the gastrointestinal tract of the candidate mammal, e.g. by noninvasive procedures including fiberoptic endoscopy, magnetic resonance imaging (MRI) and radiographic methods such as double-contrast barium x-ray. Other clinical symptomology and signs would include those gained from direct examination of the gastric mucosa of the candidate mammal, for example by means of biopsy.

### BRIEF DESCRIPTION OF THE DRAWINGS

- **Figure 1:**: Effect of ML 3000 on pig microsomal H,K-ATPase activity;
- **Figure 2:**: Effect of omeprazole on pig microsomal H,K-ATPase activity;
- **Figure 3:**: Effect of pH on ML 3000 inhibition of gastric H,K-ATPase;
- **Figure 4:**: Effect of ML 3000 dilution on inhibition of gastric H,K-ATPase;
- **Figure 5:**: Effect of arachidonic acid on pig microsomal H,K-ATPase activity.
- **Figure 6:**: Effect of PGE₂ on pig microsomal H,K-ATPase activity;
- **Figure 7:**: Effect of TEDBC on pig microsomal H,K-ATPase activity;
- **Figure 8:**: Effect of ZD-2138 on pig microsomal H,K-ATPase activity;
- **Figure 9:**: Effect of acetyl salicylic acid on pig microsomal H,K-ATPase activity.
- **Figure 10:**: Effect of NS 398 on pig microsomal H,K-ATPase activity;
- **Figure 11:**: Effect of naproxen on pig microsomal H,K-ATPase activity;
- **Figure 12:**: Effect of indomethacin on pig microsomal H,K-ATPase activity;
- **Figure 13:**: Effect of leukotriene B4 on pig microsomal H,K-ATPase activity;
- **Figure 14:**: Effect of leukotriene D4 on gastric H,K-ATPase;
- **Figure 15:**: Effect of ML 3000 on histamine-stimulated acidification in rabbit gastric glands;
- **Figure 16:**: Effect of ML 3000 on forskolin-stimulated acidification in rabbit gastric glands;
- **Figure 17:**: Effect of ML 3000 on baseline IL-8 secretion by human gastric epithelial cells;
- **Figure 18:**: Effect of ML 3000 on IL-8 secretion by IL-1β-stimulated human gastric epithelial cells;
- **Figure 19:**: Effect of ZD 2138 on baseline IL-8 secretion by human gastric epithelial cells;
- **Figure 20:**: Effect of ZD 2138 on IL-8 secretion by IL-1β-stimulated human gastric epithelial cells.

### DESCRIPTION OF PREFERRED EMBODIMENTS

In order to further demonstrate the methods and compositions of the present invention, there is presented in the paragraphs which follow specific descriptive examples of typical procedures which may be employed in carrying out said methods. Said examples are intended to be illustrative.

### EXAMPLE 1

Effects of ML 3000 on gastric microsomal K⁺-stimulated, SCH28080-sensitive H,K-ATPase activity.

Strategy. Microsomal gastric H,K-ATPase was prepared from pig gastric mucosal homogenates by differential centrifugation. Briefly, pig stomachs obtained at a slaughter-house within 1 hour post mortem were washed with ice-cold 0,25 M sucrose and the fundus was dissected from the cardiac and antral regions. All subsequent prodedures were at 4 °C. The mucosa was flooded with saturated NaCl, and the surface mucus and superficial cells wiped off with paper towels. The mucosa was scraped from the underlying connective tissue, suspended (10 % w/v) in isolation buffer (0,25 M sucrose, 20 mM HEPES, pH 7,4, 1 mM EDTA, 1 mM phenylmethylsulfonyl fluoride), and disrupted by two 10 second bursts at maximum power in a Tissumizer (Tekmar, Cincinnati, OH). The homogenate was centrifuged at 20,000 g for 30 minutes, and the supernatant was centrifuged at 10⁵ g for 1 hour. The resulting microsomal pellet was resuspended in isolation buffer, and applied to a discontinuous gradient of 7 % w/v Ficoll and 34 % sucrose (both in isolation buffer). After 3 hours at 32,500 rpm (Sorvall AH 629 rotor), the microsomal band (G1) recovered from the 7 % Ficoll interface was resuspended to 10 mg/ml in 15 mM PIPES-Tris, pH 6,8, diluted 1:1 with cold 60 % sucrose, lyophilized in 0,5 ml aliquots, and stored at -70 °C. Gl microsomes were about 0,1 microns in diameter, were enriched in H.K.-ATPase activity, and more than 80 % of their protein content banded at 94 kDa by SDS-PAGE. ATP hydrolytic activity of microsomes was quantitated in terms of inorganic phosphate release from substrate ATP and measured in graded concentrations (ranging from 10⁻⁹M to 10⁻⁴M) of ML 3000, and a wide range of other compounds. Reaction mixtures for assay of K⁺-stimulated ATPase activity in pig gastric microsomal membranes contained 5 µg membrane protein, 100 mM Tris-acetate, pH 7.0, 1mM MgCl₂, 1 mM NaNa, 0,1 mM EGTA, 5 µM ATP (γ-³²P-ATP, 10 Ci/mmol, NEN, Boston, MA), 0-10 mM KCI, and 0-100 µM SCH 28080. After 20 minutes incubation at 37°C, reactions were stopped by addition of 10 % w/v activated charcoal (Sigma), 5,5 % w/v trichloroacetic acid, vortexed vigorously, and centrifuged at 14,000 g for 10 minutes at 4 °C. Inorganic phosphate (γ-³²Pi) content of the supernatant was measured by scintillation counting. Specific H,K-ATPase activity was calculated as the difference in microsomal ATPase activities in the presence and absence of the specific gastric H,K-ATPase inhibitor SCH28080, and was expressed as mmoles Pi/mg protein/hr; graphical depiction of the data shows percent inhibition of H,K-ATPase activity as a function of compound concentrations. Graphs with standard error bars present inter-assay data variance in three independent assays in each of which ATPase activities in three separate but identical reaction conditions were measured. Graphs without standard error bars represent the mean ATPase activities in three separate but identical reaction conditions in one of at least three independent assays; typical data are shown in these cases.

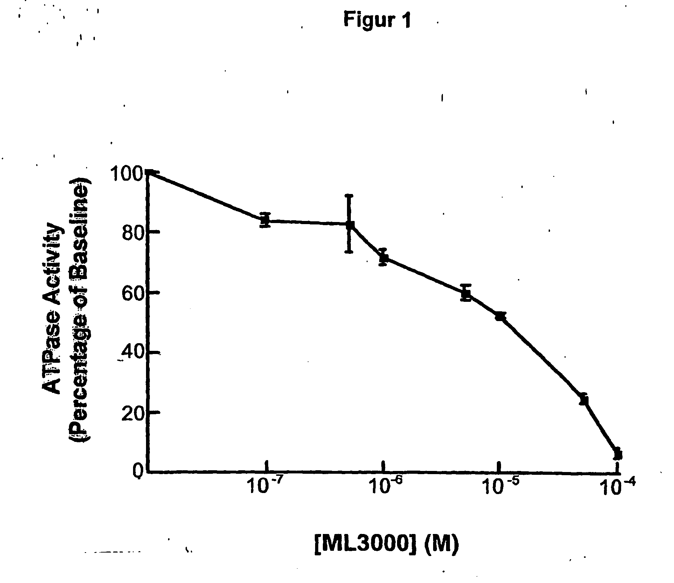

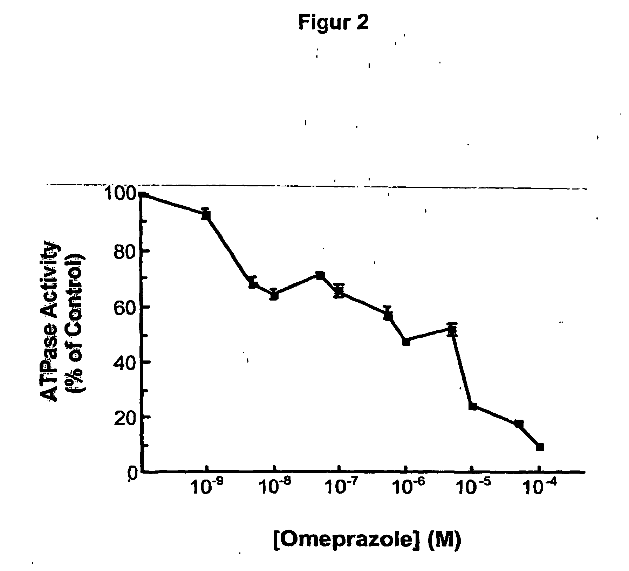

Results. Gastric H,K-ATPase activity was dose-dependently inhibited by ML 3000, with a half-maximal inhibitory concentration (IC₅₀) of 15 µM (Figure 1). The inhibitory activity of ML 3000 was compared to that of a classical proton pump inhibitor (PPI), the substituted benzimidazole omeprazole. Figure 2 shows the effect of omeprazole on H,K-ATPase activity under assay conditions identical to those in Figure 1; the estimated IC₅₀ for omeprazole was 1 µM. These data show that ML 3000 and omeprazole display comparable IC₅₀ with respect to gastric H,K-ATPase activity, at least in the setting of this particular in vitro assay under the specified conditions. Published IC₅₀ for omeprazole with respect to gastric proton pump activity range from 470 nM to 36 µM depending on the conditions of the assay (3,4,5). For other PPls, picoprazole IC₅₀ is 2 µM (6), rabeprazole IC₅₀ is 72 nM (3), and lansoprazole IC₅₀ is 2.1 µM (7).

The wide range of published PPI IC₅₀ values for microsomal H,K-ATPase reflects the mechanistic necessity for compound acidification to allow formation of a thiol-reactive sulfoxide intermediate which then irreversibly derivatizes H,K-ATPase a subunit cysteine residues leading to enzyme inhibition. Omeprazole at neutral or higher pH exerts no inhibitory effects on gastric H,K-ATPase. Microsomal vesicle preparations vary widely in their ion-tightness, which affects the extent to which internal pH can be lowered by H,K-ATPase turnover, and therefore the extent to which omeprazole diffusing into the vesicle can be acidified and activated. Alternatively, prior in vitro acidification of omeprazole ensures induction of its inhibitory properties, and is essential in assays carried out using ion-permeable microsomal H,K-ATPase preparations. For this reason, the omeprazole inhibitory data shown in Figure 2 were derived using omeprazole acidified to pH 6.1 and incubated with the enzyme for 30 min at the same pH (8).

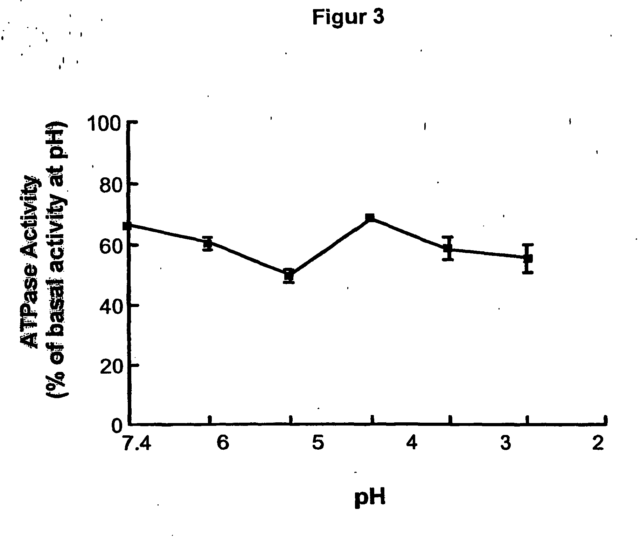

To determine whether ML 3000 displayed comparable acid-activation properties, a half-maximal inhibitory concentration of ML 3000 was titrated to different pHs and the effects on H,K-ATPase activity were measured. As shown in Figure 3, acidification of ML 3000 had no significant effect on its H,K-ATPase inhibitory profile. These data indicate that although ML 3000 and omeprazole have comparable IC₅₀ for H,K-ATPase, ML 3000 unlike omeprazole does not require acidification for induction of inhibitory activity.

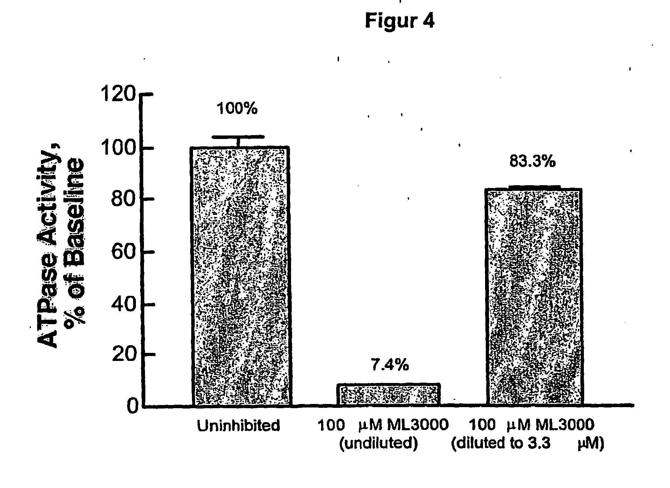

Given that PPls are irreversible inhibitors of H,K-ATPase activity, covalently binding to the catalytic α-subunit, it was determined whether ML 3000 inhibition of H,K-ATPase was reversible or irreversible. Gastric H,K-ATPase-enriched microsomes were treated with a maximally-inhibitory concentration of ML 3000 and then diluted with a large excess of buffer to reduce the ML 3000 concentration from 100 µM to 3.3 µM. The results, shown in Figure 4, indicated that dilution of ML 3000 restored H,K-ATPase activity, and are consistent with ML 3000 inhibiting H,K-ATPase activity in a reversible manner, ie., ML 3000 does not covalently derivatize either sub-unit of the gastric H,K-ATPase in vitro.

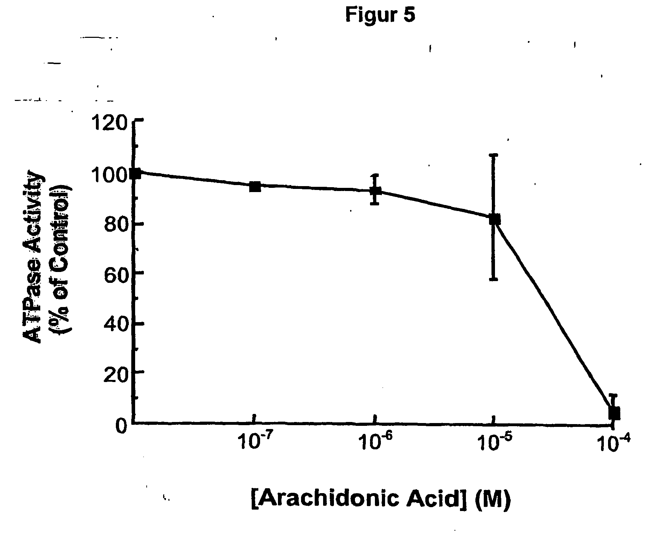

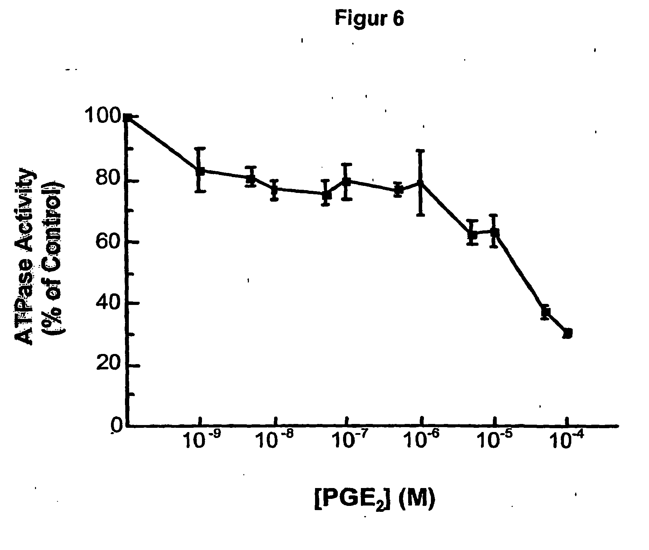

Arachidonic acid and prostaglandin E2 (PGE₂) also dose-dependently inhibited H,K-ATPase activity, with IC₅₀ of 30 µM and 45 µM respectively (Figures 5 and 6). The PGE2 data contradict a previous study in which no inhibitory effect of PGE₂ on pig gastric H,K-ATPase (9) was found. Differences in the specific ATPase assay used in that study may account for this discrepancy. Since ML3000 and arachidonic acid are anionic amphiphiles, their inhibitory effects could result from specific interactions with H,K-ATPase sub-unit binding sites, or from less-specific hydrophobic interactions with H,K-ATPase-associated microsomal membrane lipids, or a combination of both factors.

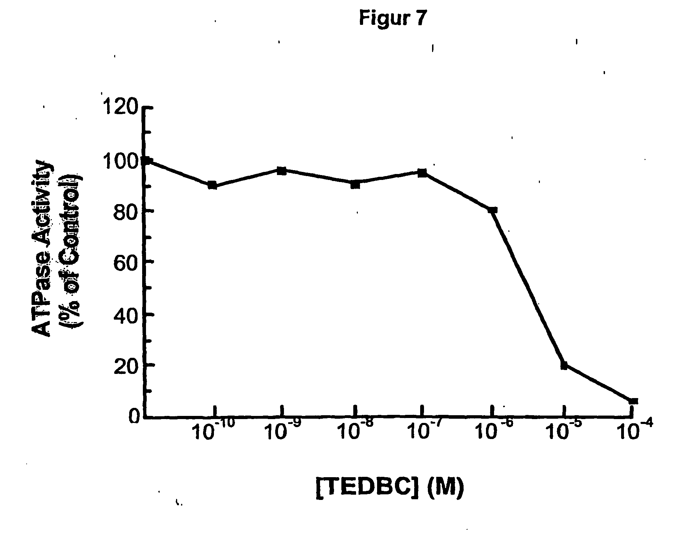

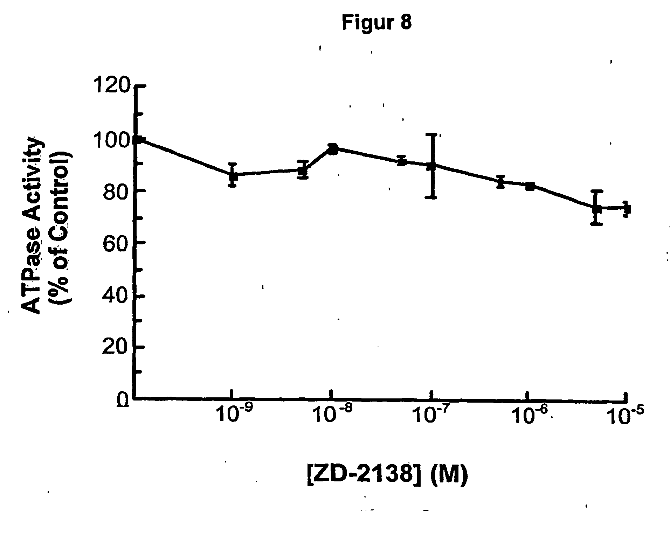

Since ML 3000 also shows 5-lipoxygenase inhibition, the effects of two lipoxygenase inhibitors on microsomal H,K-ATPase activity were studied. Figure 7 shows that 2-(1-thienyl)ethyl 3,4-dihydroxybenzylidenecyanoacetate (TEDBC), a powerful inhibitor of 5-, 12-, and 15-lipoxygenses, inhibited microsomal H,K-ATPase activity with an IC₅₀ of 3.3 µM. In contrast, the 5-lipoxygenase-specific inhibitor 6-((3-fluor-5-(methoxy-3,4,5,6-tetrahydro-2H-pyran-4-yl)phenoxy)methyl)chinolin (ZD-2138) had a minimal effect on H,K-ATPase activity (~20% inhibition at 10⁻⁶ M) (Figure 8). These data are consistent with gastric microsomal 12- and 15-lipoxygenases playing a role in H,K-ATPase activation, or with direct interaction of TEDBC with H,K-ATPase subunits altering enzyme conformation and hence activity.

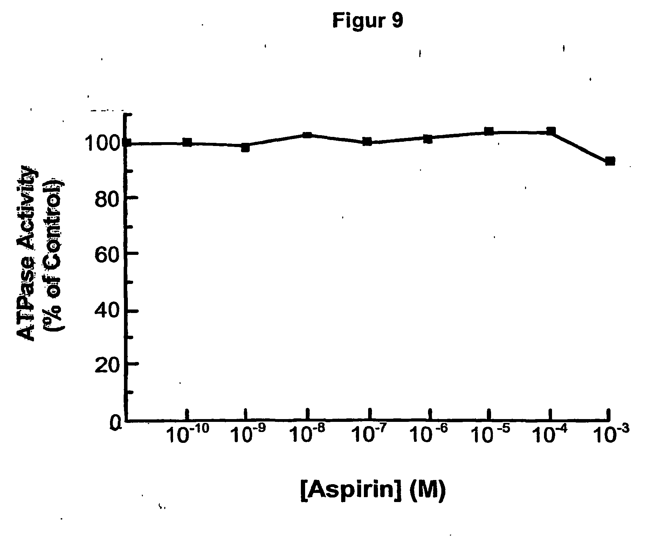

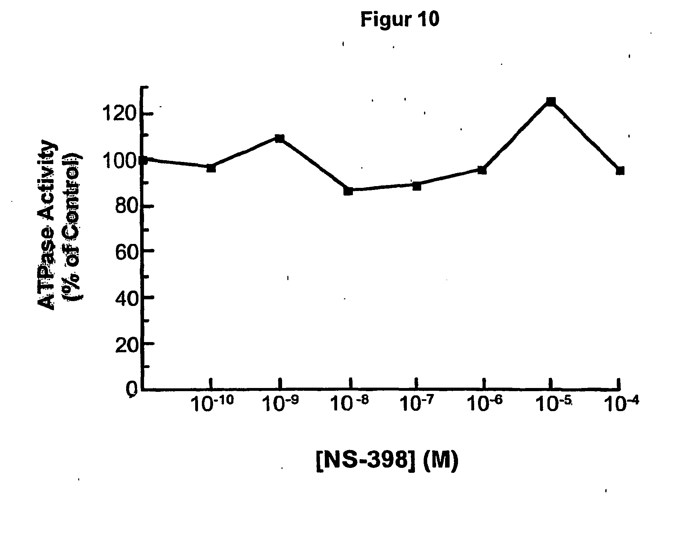

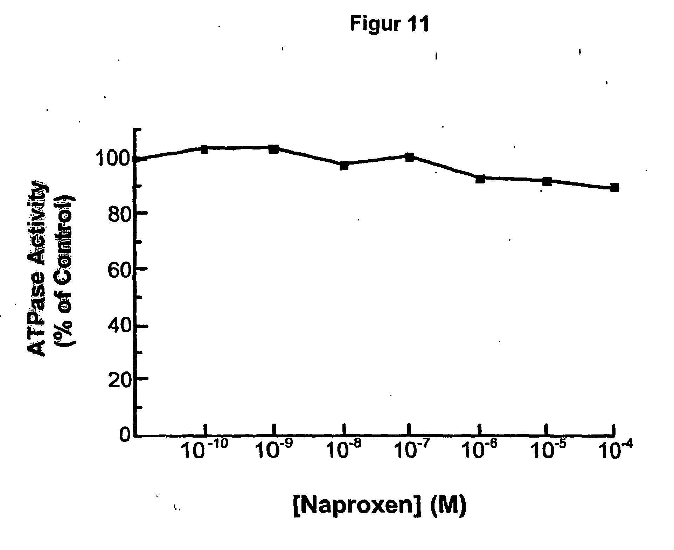

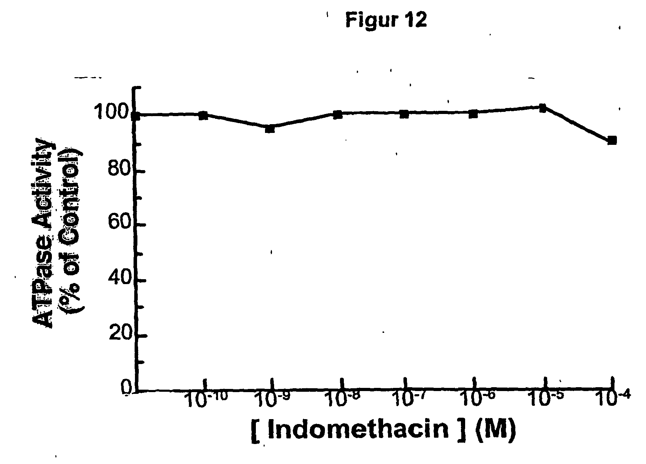

In order to compare the H,K-ATPase inhibitory effect of ML 3000 with other NSAIDs, the effects of acetyl salicylic acid, naproxen, indomethacin and a selective COX-2 inhibitor, NS 398, on proton pump activity were measured. All four NSAIDs were without inhibitory effects on microsomal H,K-ATPase activity at concentrations up to 10⁻⁴ M (10⁻³ M in the case of acetyl salicylic acid) (Figures 9, 10, 11 and 12). Indomethacin was previously reported to inhibit gastric H,K-ATPase at somewhat higher concentration (Ki = 0.67 x 10⁻³ M) (10). This data clearly differentiate ML 3000 from other NSAIDs in terms of inhibitory effect on gastric H,K-ATPase activity.

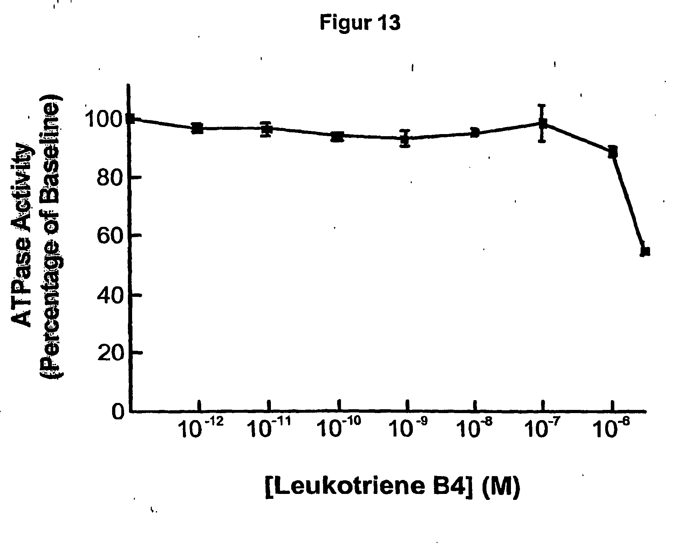

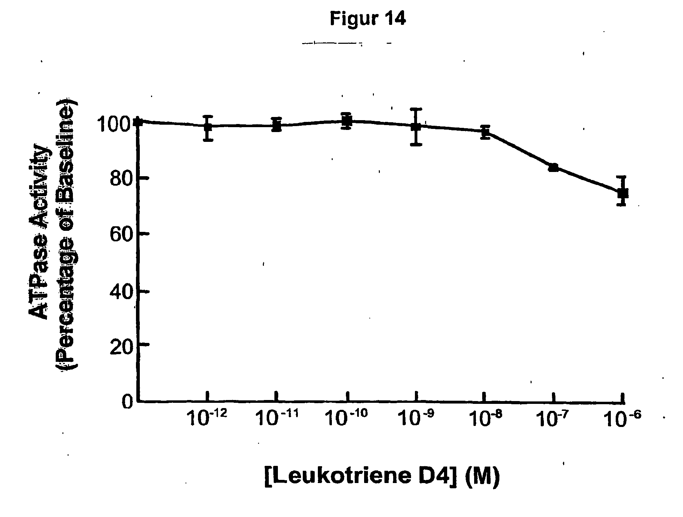

In order to establish whether the ML 3000 inhibition of gastric H,K-ATPase reflected the compound's effects on putative functional leukotriene metabolic pathways present in pig gastric microsomes, the effects of leukotriene B4 and D4 on H,K-ATPase activity were studied. Solubility issues precluded studying LTB4 or LTD4 concentrations greater than 1 µM. As shown in Figures 13 and 14, neither leukotriene showed any inhibitory activity against H,K-ATPase at physiological concentrations (10⁻⁹-10⁻⁸ M); only at non-physiological concentrations greater than 10⁻⁷ M was there any significant attentuation of H,K-ATPase activity.

### EXAMPLE 2

Effects of ML 3000 and other compounds on gastric parietal cell histamine-stimulated acid accumulation.

Strategy. Gastric parietal cells were isolated from New Zealand White Rabbits by pronase/collagenase digestion of fundic mucosa followed by enrichment of cells on discontinuous Nycodenz gradients in a manner known per se. Aminopyrine accumulation into parietal cells was assessed in 96 well filter plates with Durapore membranes. Briefly, cells were preincubated with [¹⁴C]-aminopyrine and then 100,000 cells/200 µl per well were incubated without or with test compounds for 15 minutes prior to incubation for a further 30 min in the absence or presence of 100 µM histamine. All determinations were performed in quadruplicate. Basal aminopyrine accumulation was determined as aminopyrine accumulation into untreated cells subtracted from accumulation in the presence of KSCN (a reflection of non-specific isotope trapping). Graphical depiction of the data shows percent inhibition of histamine-stimulated acid accumulation by the cells as a function of compound concentrations.

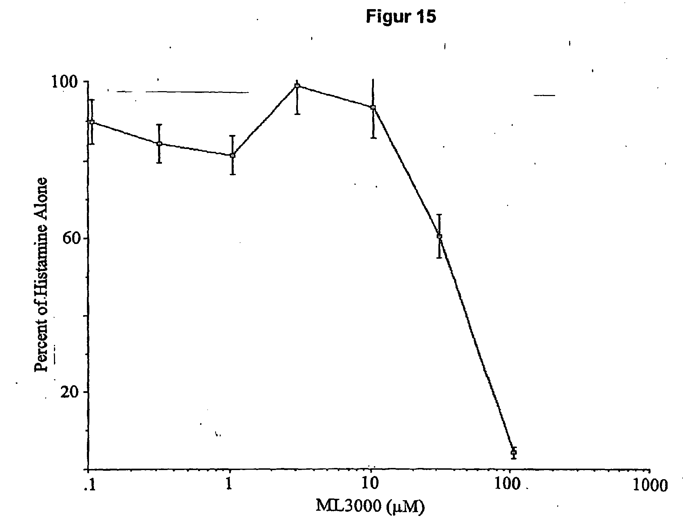

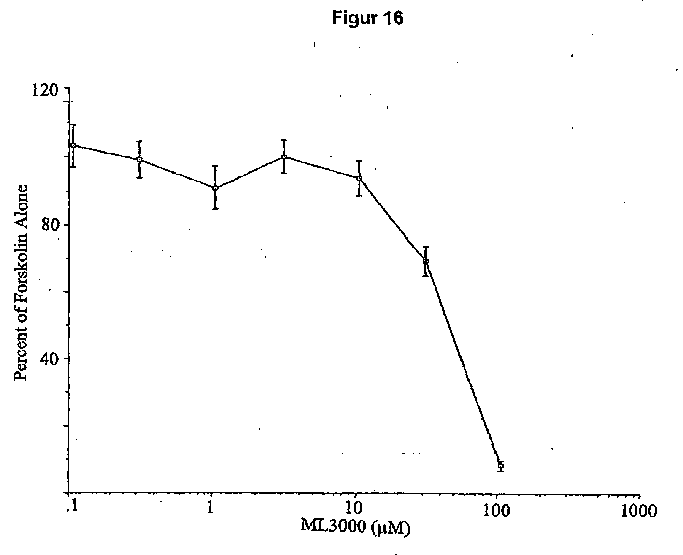

Results. ML 3000 dose-dependently inhibited histamine-stimulated acid accumulation by rabbit gastric parietal cells, with a half-maximal inhibitory concentration (IC₅₀) of 40 µM (Figure 15). ML 3000 also dose-dependently inhibited forskolin-stimulated acid accumulation by rabbit gastric parietal cells, with a half-maximal inhibitory concentration (IC₅₀) of ~45 µM (Figure 16). These data indicate that ML 3000 affects parietal cell acid-secretory mechanisms downstream of cAMP mobilization induced by histamine H2 receptor activation. The data are consistent with ML 3000 inhibition of parietal cell acid secretion resulting from direct interaction of ML 3000 with the gastric H,K-ATPase. However, the discepancy between ML 3000 IC₅₀ in microsomal vesicles (15 µM) and in isolated parietal cells (40-45 µM) suggests that ML 3000 access to the intracellular H,K-ATPase compartment in parietal cells may be slowed by permeability constraints at the plasma membrane.

Also, as was found with microsomal H,K-ATPase, other NSAIDs such as acetyl salicylic acid, naproxen, and NS 398 (up to concentrations of 10⁻⁴ M) had no effect on acid accumulation by isolated rabbit parietal cells.

### EXAMPLE 3

Effects of ML 3000 on IL-1β-induced and Heliobacter pylori-induced IL-8 secretion in human gastric adenocarcinoma (AGS) cells.

Strategy. AGS cells were incubated with test compounds, challanged with IL-1β, and sebsequent secretion of IL-8 into the culture medium was measured by enzyme linked immunosorbent assay; graphical depiction of the data shows precent inhibition od unstimulated or stimulated IL-8 secrretion as a function of a compound concentrations.

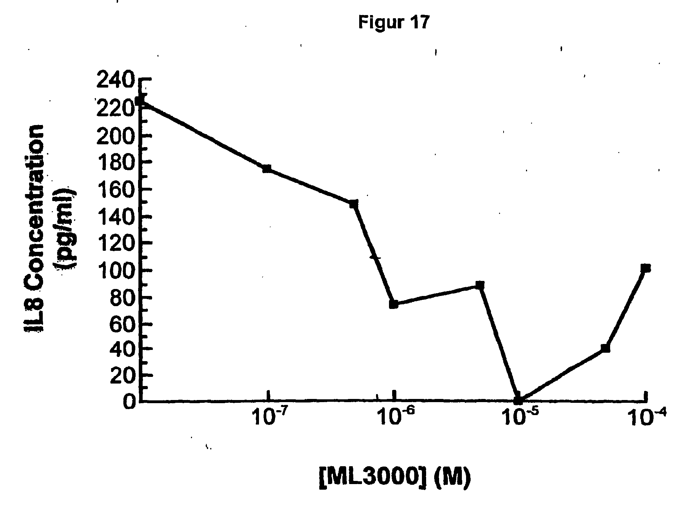

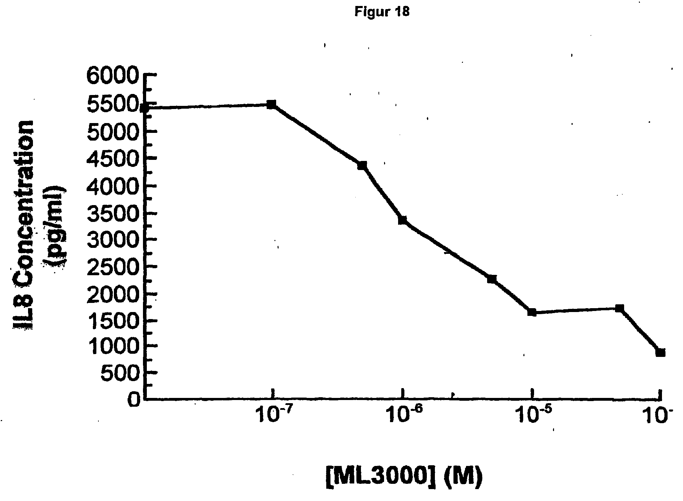

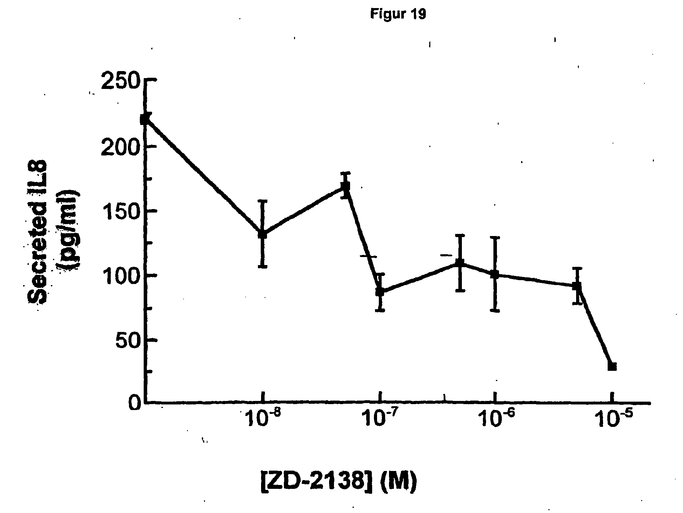

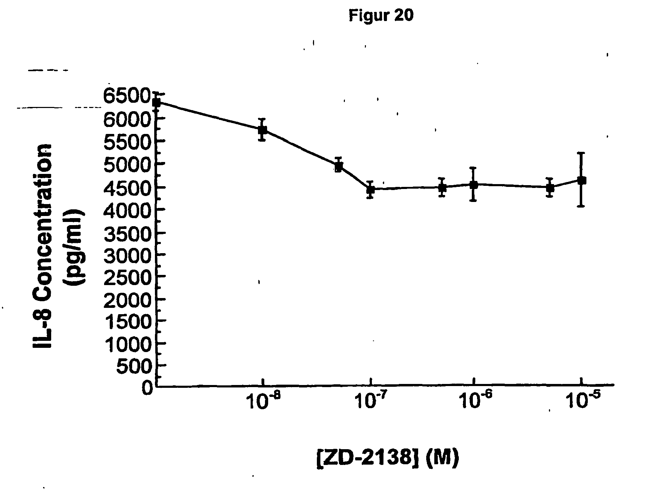

Results. Without stimulation by IL-1β, and in the absence of ML3000 or ZD2138, AGS cells (5 x 104 in µl culture medium) secreted IL-8 over a period of 6 hr to a concentration of ~225 pg/ml (Figures 17 and 19). When stimulated by IL-1β (20 ng/ml), AGS cell IL-8 secretion over a period of 6 hr was increased ~27-fold, to a concentration of -6000 pg/ml (Figures 18 and 20). ML3000 inhibited both baseline (Figure 17) and IL-1β-stimulated IL-8 secretion (Figure 18), with IC₅₀ of 0.75 µM and 30 µM repectively.

The 5-lipoxygenase-specific inhibitor (ZD-2138), which was withour effect on microsomal H⁺,K⁺-ATPase activity, showed a dose-dependent inhibition of baseline IL-8 secretion by AGS cells (Figures 19), with an IC50 of -0.4 µM. In contrast, ZD-2138 was without effect on IL-1β-stimulated IL-8 secretion by AGS cells (Figure 20). H⁺,K⁺-ATPase inhibition by ML3000, which was demonstrated above, does not underlie IL-8 secretory inhibtion in this model since AGS cells do not express H⁺,K⁺-ATPase.

To the extent that IL-8 is a potent inflammatory mediator in the gastric mucosa, the finding that ML3000 profoundly inhibits baseline and IL-1β-stimulated IL-8 secretion in gastric epithelial cells suggests the inhibtion is not effected by the 5-lipoxygenase inhibitory activity of ML3000.

### REFERENCES

1. Laufer S, Tries S, Augustin J, Dannhardt G. Pharmacological profile of a new pyrrolizine derivative inhibiting the enzymes cyclo-oxygenase and 5-lipoxygenase. Arzneimittelforschung 1994;44:629-36.
2. Laufer S, Tries S, Augustin J, Elsasser R, Albrecht W, Guserle R, et al. Acute and chronic anti-inflammatory properties of [2,2-dimethyl-6-(4- chlorophenyl)-7-phenyl-2,3-dihydro-1H-pyrrolizine-5-yl]-acetic acid. Arzneimittelforschung 1995;45:27-32.
3. Morii M, Takata H, Fujisaki H, Takeguchi N (1990) The potency of substituted benzimidazoles such as E3810, omeprazole, Ro 18-5364 to inhibit gastric H,K-ATPase is correlated with the rate of acid-inactivation of the inhibitor. Biochem Pharmacol, 39(4):661-667;
4. Beil W, Sewing KF (1984) Inhibition of partially-purified H,K-ATPase from guinea-pig isolated and enriched parietal cells by substituted benzimidazoles. Br J Pharmacol 82(3):651-657;
5. Keeling DJ, Fallowfield C, Milliner KJ, Tingley SK, Ife RJ, Underwood AH (1985) Studies on the mechanism of action of omeprazole. Biochem Pharmacol, 34(16):2967-2973);
6. Wallmark B, Sachs G, Mardh S, Fellenius E. Inhibition of gastric H,K-ATPase by the substituted benzimidazole, picoprazole. Biochim Biophys Acta, 728:31-38,1983
7. Nagaya H, Inatomi N, Nohara A, Satoh H (1991) Effects of the enantiomers of lansoprazole (AG-1749) on H, K-ATPase activity in canine gastric microsomes and acid formation in isolated canine parietal cells. Biochem Pharmacol, 42(10):1875-1878;
8. Wallmark B, Jaresten B-M, Larsson H, Ryberg B, Brandstrom A, Fellenius E (1983a) Differentiation among inhibitory actions of omeprazole, cimetidine, and SCN- on gastric acid secretion. Am J Physiol 245 (Gastrointest Liver Physiol 8): G64-G71 ;
9. Im WB, Blakeman DP (1982) Inhibition of gastric H,K-ATPase by unsaturated long-chain fatty acids. Biochim Biophys Acta, 692:355-360;
10. Spenney JG and Mize KS (1977) Inhibition of gastric K-ATPase by phenylbutazone and indomethacin. Biochem Pharmacol, 26:1241-1245.
11. Wallace JL, Carter L, McKnight W, Tries S, Laufer S (1994) ML3000 reduces gastric prostaglandin synthesis without causing mucosal injury. European Journal of Pharmacology 271: 525-531.
12. Laufer S, Tries S, Augustin I, Elsäßer R, Algate DR, Atterson PR, Munt PL (1994) Gastrointestinal Tolerance of [2,2-Dimethyl-6-(4-chlorophenyl)-7-phenyl-2,3-dihydro-1H-pyrrolizine-5 yl]-acetic Acid in the Rat. Arzneim.-Forsch./Drug Res. 44 (II): 1329-1333.
13. Tries S and Laufer S (2001) The pharmacological profile of ML3000: A new pyrrolizine derivative inhibiting the enzymes cyclo-oxygenase and 5-lipoxygenase. Inflammopharmacology 9: 113-124.

## Claims

1. The use of [6-(4-chlorophenyl)-2,2-dimethyl-7-phenyl-2,3-dihydro-1H-pyrrolizine-5-yl]-acetic acid of the formula (Ia) 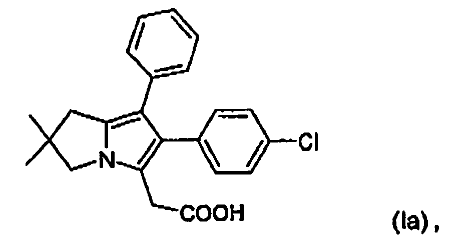 a physiologically acceptable salt or a physiologically hydrolysable ester thereof, for preparing a pharmaceutical composition for treating a gastric mucosal lesion.

2. The use of [6-(4-chloropheny)-2,2-dimethyl-7-phenyl-2,3-dihydro-1H-pymolizine-5-yl]-acetlc acid of the formula (1a) 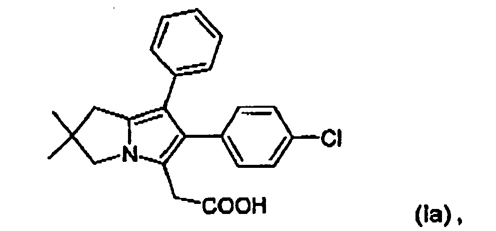 a physiologically acceptable salt or a physiologically hydrolysable ester thereof, for preparing a pharmaceutical composition for treating or preventing erosive gastritis,

3. The use of [6-(4-chlorophenyl)-2,2-dimethyl-7-phenyl-2,3-dihydro-1H-pyrrolizine-5-yl]-acetic acid of the formula (Ia) 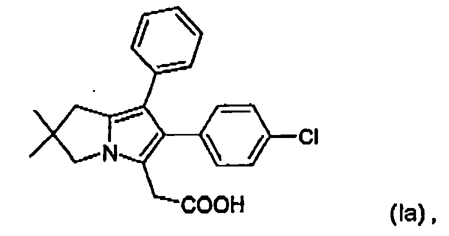 a physiologically acceptable salt or a physiologically hydrolysable ester thereof, for preparing a pharmaceutical composition for treating or preventing non-erosive gastritis.

4. The use of [6-(4-chlorophenyl)-2,2-dimethyl-7-phenyl-2,3-dihydro-1H-pyrrolizine-5-yl]-acetic acid of the formula (Ia) 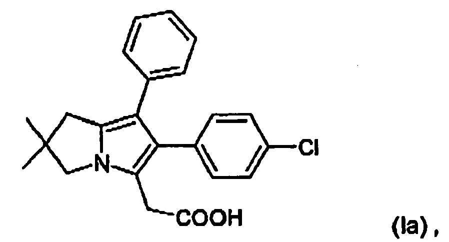 a physiologically acceptable salt or a physiologically hydrolysable ester thereof, for preparing a pharmaceutical composition for treating or preventing gastric ulceration.

5. The use of [6-(4-chlorophenyl)-2,2-dimethyl-7-phenyl-2,3-dihydro-1H-pyrrolizine-5-yl]-acetic acid of the formula (Ia) 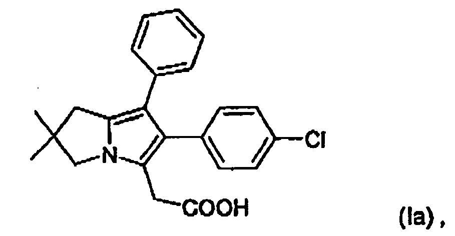 a physiologically acceptable salt or a physiologically hydrolysable ester thereof, for preparing a pharmaceutical composition for treating or preventing Ulcus duodeni or Ulcus ventricull.

6. The use according to any one of the preceding claims, including in addition to the compound of Formula (Ia), the physiologically acceptable salt or the physiologically hydrolysable ester thereof, the use of one or more members selected from the group consisting of antibacterially active agents, further gastroprotective agents such as further proton inhibitors and H₂-receptor antagonists, antacid agents, alginates and prokinetic agents.

7. A combination of
(i) [6-(4-chlorophenyl)-2,2-dimethyl-7-phenyl-2,3-dihydro-1H-pyrrolizine-5-yl]-acetic acid of the formula (Ia) 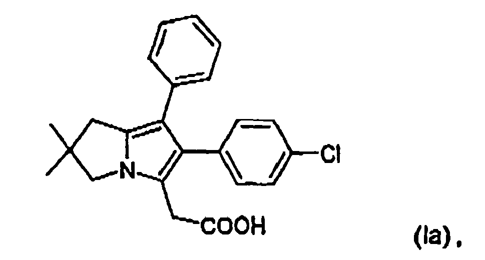 a physiologically acceptable salt or a physiologically hydrolysable ester thereof, with
(ii) one or more than one ulcerogenic anti-inflammatory agent for use in therapy, wherein the ulcerogenic anti-inflammatory agent is selected from the group consisting of acetylsalicylic acid (ASA), sodium salicylate, acetaminophen, phenacetin, ibuprofen, ketoprofen, indomethacin, flurbiprofen, diclofenac, naproxen, piroxicam, tebufelone, naburnetone, tenidap, alcofenac, antipyrine, amimopyrine, dipyrone, animopyrone, phenylbutazone, clofezone, oxyphenbutazone, prexazone, apazone, benzydamine, bucolome, cinchopen, clonixin, ditrazol, epirizole, fenoprofen, floctafenin, flufenamic acid, glaphenine, indoprofen, meclofenamic acid, mefenamic acid, niflumic acid, salidifamides, sulindac, suprofen, tolmetin, nabumetone, tiaramide, proquazone, bufexamac, flumizole, tinoridine, timegadine, dapsone, diflunisal, benorylate, fosfosal, fenclofenac, fentiazac, tilomisole, carprofen, fenbufen, oxaprozin, tiaprofenic acid, pirprofen, feprazone, piroxicam, sudoxicam, isoxicam, tenoxicam.

8. A pharmaceutical composition comprising
(i) [6-(4-chlorophenyl)-2,2-dimethyl-7-phenyl-2,3-dihydro-1H-pyrrolizine-5-yl]-acetic acid of the formula (la) 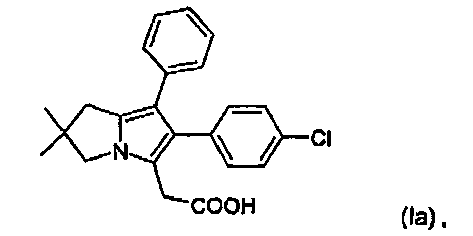 a physiologically acceptable salt or a physiologically hydrolysable ester thereof,
(ii) one or more than one ulcerogenic anti-inflammatory agent for use in therapy, and optionally
(iii) a pharmaceutically acceptable carrier,
wherein the ulcerogenic anti-inflammatory agent is selected from the group consisting of acetylsalicylic add (ASA), sodium salicylate, acetaminophen, phenacetin, ibuprofen, ketoprofen, indomethacin, flurbiprofen, diclofenac, naproxen, piroxicam, tebufelone, naburnetone, tenidap, alcofenac, antipyrine, amimopyrine, dipyrone, animopyrone, phenylbutazone, clofezone, oxyphenbutazone, prexazone, apazone, benzydamine, bucolome, cinchopen, clonixin, ditrazol, epirizole, fenoprofen, floctafenin, flufenamic acid, glaphenine, indoprofen, meclofenamic acid, mefenamic acid, niflumic acid, salidifamides, sulindac, suprofen, tolmetin, nabumetone, tiaramide, proquazone, bufexamac, flumizole, tinoridine, timegadine, dapsone, diflunisal, benorylate, fosfosal, fenclofenac, fentiazac, tilomisole, carprofen, fenbufen, oxaprozin, tiaprofenic acid, pirprofen, feprazone, piroxicam, sudoxicam, isoxicam, tenoxicam.

## Patentansprüche

1. Verwendung von [6-(4-Chlorphenyl)-2,2-dimethyl-7-phenyl-2,3-dihydro-1 H-pyrrolizin-5-yl]-essigsäure der Formel (la) 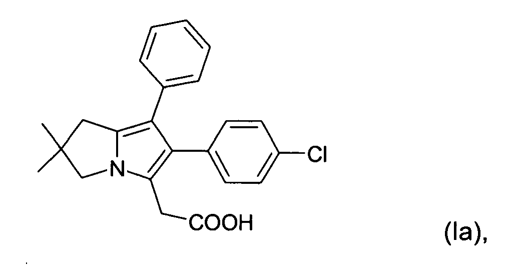 eines physiologisch akzeptablen Salzes oder eines physiologisch hydrolysierbaren Esters davon zur Herstellung einer pharmazeutischen Zusammensetzung zur Behandlung einer Magenschleimhautläsion.

2. Verwendung von [6-(4-Chlorphenyl)-2,2-dimethyl-7-phenyl-2,3-dihydro-1H-pyrrolizin-5-yl]-essigsäure der Formel (la) 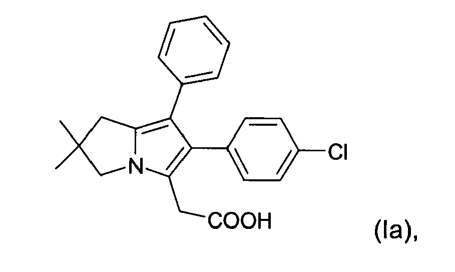 eines physiologisch akzeptablen Salzes oder eines physiologisch hydrolysierbaren Esters davon zur Herstellung einer pharmazeutischen Zusammensetzung zur Behandlung oder Vorbeugung von erosiver Gastritis.

3. Verwendung von [6-(4-Chlorphenyl)-2,2-dimethyl-7-phenyl-2,3-dihydro-1 H-pyrrolizin-5-yl]-essigsäure der Formel (la) 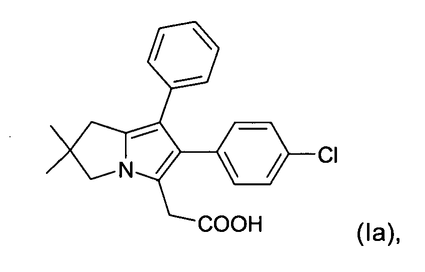 eines physiologisch akzeptablen Salzes oder eines physiologisch hydrolysierbaren Esters davon zur Herstellung einer pharmazeutischen Zusammensetzung zur Behandlung oder Vorbeugung von nicht-erosiver Gastritis.

4. Verwendung von [6-(4-Chlorphenyl)-2,2-dimethyl-7-phenyl-2,3-dihydro-1H-pyrrolizin-5-yl]-essigsäure der Formel (la) 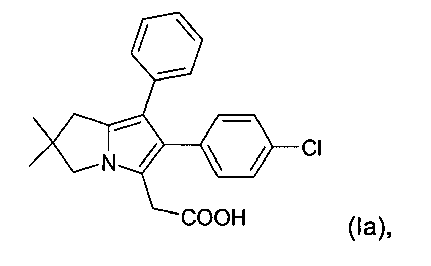 eines physiologisch akzeptablen Salzes oder eines physiologisch hydrolysierbaren Esters davon zur Herstellung einer pharmazeutischen Zusammensetzung zur Behandlung oder Vorbeugung von Magengeschwüren.

5. Verwendung von [6-(4-Chlorphenyl)-2,2-dimethyl-7-phenyl-2,3-dihydro-1H-pyrrolizin-5-yl]-essigsäure der Formel (la) 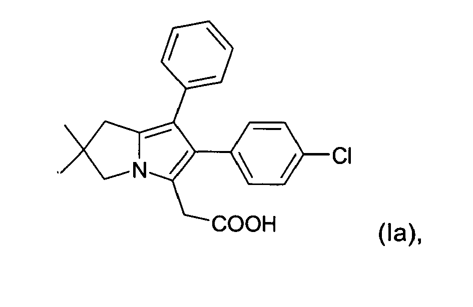 eines physiologisch akzeptablen Salzes oder eines physiologisch hydrolysierbaren Esters davon zur Behandlung oder Vorbeugung von Ulcus duodeni oder Ulcus ventriculi.

6. Verwendung nach einem der vorhergehenden Ansprüche, umfassend zusätzlich zu der Verbindung der Formel (Ia), dem physiologisch akzeptablen Salz und dem physiologisch hydrolysierbaren Ester davon die Verwendung von einem oder mehreren Mitgliedern ausgewählt unter antibakteriell wirksamen Mitteln, weiteren gastroprotektiven Mitteln, wie z.B. weiteren Protonenhemmern und H₂-Rezeptorantagonisten, Antazida, Alginaten und prokinetischen Mitteln.

7. Kombination von
(i) [6-(4-Chlorphenyl)-2,2-dimethyl-7-phenyl-2,3-dihydro-1 H-pyrrolizin-5-yl]-essigsäure der Formel (la) 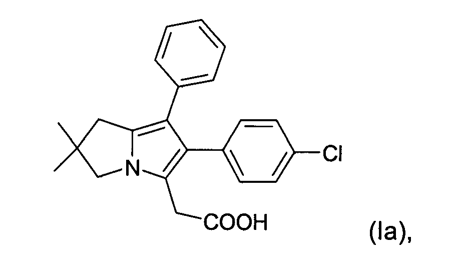 eines physiologisch akzeptablen Salzes oder eines physiologisch hydrolysierbaren Esters davon
mit
(ii) einem oder mehr als einem ulcerogenen antiinflammatorischen Mittel zur Verwendung in der Therapie, wobei das ulcerogene antiinflammatorische Mittel ausgewählt ist unter Acetylsalicylsäure (ASA), Natriumsalicylat, Acetaminophen, Phenacetin, Ibuprofen, Ketoprofen, Indomethacin, Flurbiprofen, Diclofenac, Naproxen, Piroxicam, Tebufelon, Nabumeton, Tenidap, Alcofenac, Antipyrin, Amimopyrin, Dipyron, Animopyron, Phenylbutazon, Clofezon, O-xyphenbutazon, Prexazon, Apazon, Benzydamin, Bucolom, Cinchopen, Clonixin, Ditrazol, Epirizol, Fenoprofen, Floctafenin, Flufenamsäure, Glaphenin, Indoprofen, Meclofenamsäure, Mefenamsäure, Niflumsäure, Salidifamiden, Sulindac, Suprofen, Tolmetin, Nabumeton, Tiaramid, Proquazon, Bufexamac, Flumizol, Tinoridin, Timegadin, Dapson, Diflunisal, Benorylat, Fosfosal, Fenclofenac, Fentiazac, Tilomisol, Carprofen, Fenbufen, Oxaprozin, Tiaprofensäure, Pirprofen, Feprazon, Piroxicam, Sudoxicam, Isoxicam, Tenoxicam.

8. Pharmazeutische Zusammensetzung, enthaltend
(i) [6-(4-Chlorphenyl)-2,2-dimethyl-7-phenyl-2,3-dihydro-1H-pyrrolizin-5-yl]-essigsäure der Formel (la) 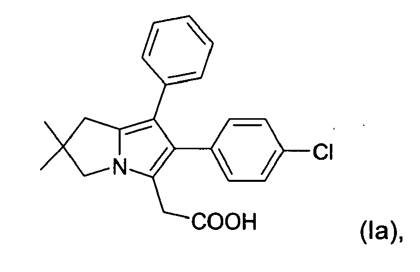 ein physiologisch akzeptables Salz oder einen physiologisch hydrolysierbaren Ester davon,
(ii) ein oder mehr als ein ulcerogenes antiinflammatorisches Mittel zur Verwendung in der Therapie, und gegebenenfalls
(iii) einen pharmazeutisch akzeptablen Träger,
wobei das ulcerogene antiinflammatorische Mittel ausgewählt ist unter Acetylsalicylsäure (ASA), Natriumsalicylat, Acetaminophen, Phenacetin, Ibuprofen, Ketoprofen, Indomethacin, Flurbiprofen, Diclofenac, Naproxen, Piroxicam, Tebufelon, Nabumeton, Tenidap, Alcofenac, Antipyrin, Amimopyrin, Dipyron, Animopyron, Phenylbutazon, Clofezon, Oxyphenbutazon, Prexazon, Apazon, Benzydamin, Bucolom, Cinchopen, Clonixin, Ditrazol, Epirizol, Fenoprofen, Floctafenin, Flufenamsäure, Glaphenin, Indoprofen, Meclofenamsäure, Mefenamsäure, Niflumsäure, Salidifamiden, Sulindac, Suprofen, Tolmetin, Nabumeton, Tiaramid, Proquazon, Bufexamac, Flumizol, Tinoridin, Timegadin, Dapson, Diflunisal, Benorylat, Fosfosal, Fenclofenac, Fentiazac, Tilomisol, Carprofen, Fenbufen, Oxaprozin, Tiaprofensäure, Pirprofen, Feprazon, Piroxicam, Sudoxicam, Isoxicam, Tenoxicam.

## Revendications

1. Utilisation de l'acide [6-(4-chlorophényl)-2,2-diméthyl-7-phényl-2,3-dihydro-1H-pyrrolizin-5-yl]-acétique de formule (Ia) 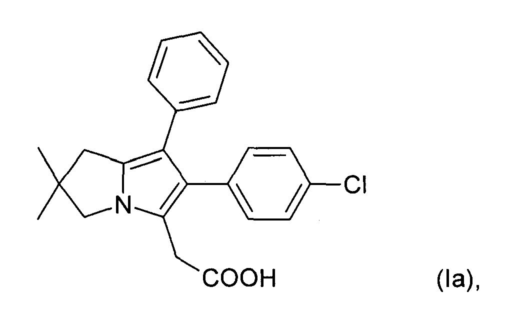 d'un sel physiologiquement acceptable ou d'un ester physiologiquement hydrolysable de celui-ci, pour préparer une composition pharmaceutique pour traiter une lésion muqueuse gastrique.

2. Utilisation de l'acide [6-(4-chlorophényl)-2,2-diméthyl-7-phényl-2,3-dihydro-1H-pyrrolizin-5-yl]-acétique de formule (Ia) 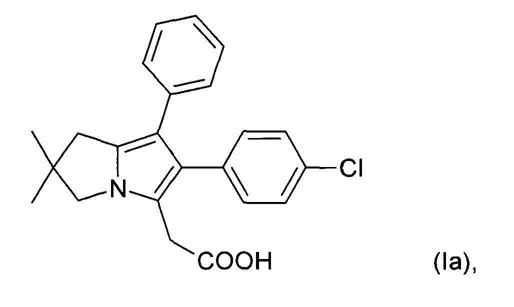 d'un sel physiologiquement acceptable ou d'un ester physiologiquement hydrolysable de celui-ci, pour préparer une composition pharmaceutique pour traiter ou prévenir une gastrite érosive.

3. Utilisation de l'acide [6-(4-chlorophényl)-2,2-diméthyl-7-phényl-2,3-dihydro-1H-pyrrolizin-5-yl]-acétique de formule (Ia) 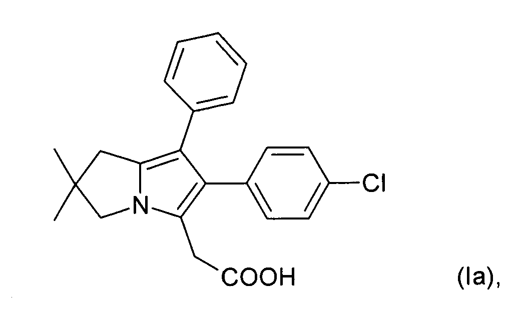 d'un sel physiologiquement acceptable ou d'un ester physiologiquement hydrolysable de celui-ci, pour préparer une composition pharmaceutique pour traiter ou prévenir une gastrite non érosive.

4. Utilisation de l'acide [6-(4-chlorophényl)-2,2-diméthyl-7-phényl-2,3-dihydro-1H-pyrrolizin-5-yl]-acétique de formule (Ia) 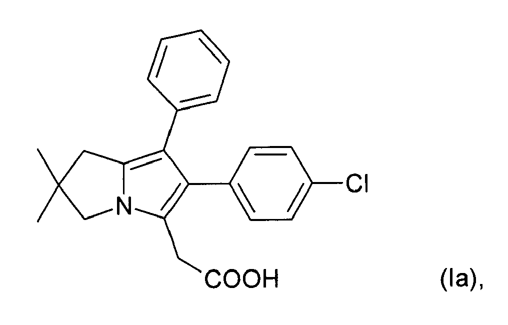 d'un sel physiologiquement acceptable ou d'un ester physiologiquement hydrolysable de celui-ci, pour préparer une composition pharmaceutique pour traiter ou prévenir l'ulcération gastrique.

5. Utilisation de l'acide [6-(4-chlorophényl)-2,2-diméthyl-7-phényl-2,3-dihydro-1H-pyrrolizin-5-yl]-acétique de formule (Ia) 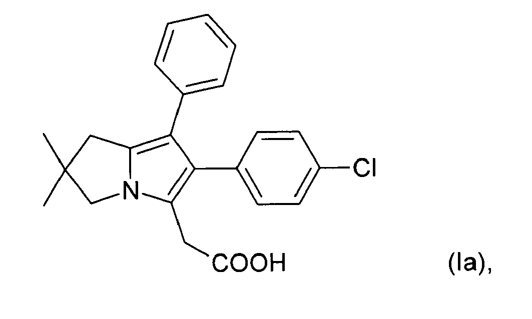 d'un sel physiologiquement acceptable ou d'un ester physiologiquement hydrolysable de celui-ci, pour préparer une composition pharmaceutique pour traiter ou prévenir l'ulcère duodénal ou l'ulcère gastrique.

6. Utilisation selon l'une quelconque des revendications précédentes, incluant, en plus du composé de formule (Ia), de son sel physiologiquement acceptable ou de son ester physiologiquement hydrolysable, l'utilisation d'un ou plusieurs membres choisis dans le groupe consistant en les agents actifs du point de vue antibactérien, d'autres agents gastroprotecteurs comme d'autres inhibiteurs des protons et des antagonistes de récepteurs H₂, des agents antiacides, des alginates et des agents procinétiques.

7. Combinaison de
(i) l'acide [6-(4-chlorophényl)-2,2-diméthyl-7-phényl-2,3-dihydro-1H-pyrrolizin-5-yl]-acétique de formule (Ia) 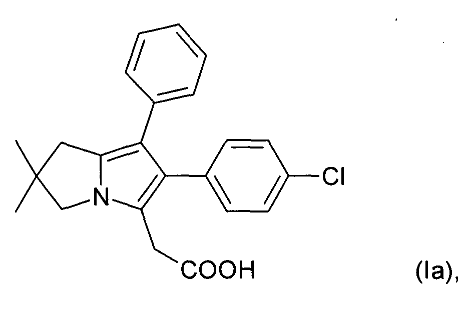 d'un sel physiologiquement acceptable ou d'un ester physiologiquement hydrolysable de celui-ci, avec
(ii) un ou plusieurs agents anti-inflammatoires ulcérogènes destinés à être utilisés en thérapie, où l'agent anti-inflammatoire ulcérogène est choisi dans le groupe consistant en l'acide acétylsalicylique (ASA), le salicylate de sodium, l'acétaminophène, la phénacétine, l'ibuprofène, le kétoprofène, l'indométhacine, le flurbiprofène, le diclofénac, le naproxène, le piroxicam, la tébufélone, la nabumétone, le ténidap, l'alcofénac, l'antipyrine, l'aminopyrine, la dipyrone, l'aminopyrone, la phénylbutazone, la clofézone, l'oxyphenbutazone, la prexazone, l'apazone, la benzydamine, le bucolome, le cinchopen, la clonixine, le ditrazol, l'épirizole, le fénoprofène, la floctafénine, l'acide flufénamique, la glaphénine, l'indoprofène, l'acide méclofénamique, l'acide méfénamique, l'acide niflumique, les salidifamides, le sulindac, le suprofène, la tolmétine, la nabumétone, le tiaramide, la proquazone, le bufexamac, le flumizole, la tinoridine, la timégadine, la dapsone, le diflunisal, le bénorylate, le fosfosal, le fenclofénac, le fentiazac, le tilomisole, le carprofène, le fenbufène, l'oxaprozine, l'acide tiaprofénique, le pirprofène, la féprazone, le piroxicam, le sudoxicam, l'isoxicam, le ténoxicam.

8. Composition pharmaceutique comprenant
(i) l'acide [6-(4-chlorophényl)-2,2-diméthyl-7-phényl-2,3-dihydro-1H-pyrrolizin-5-yl]-acétique de formule (Ia) 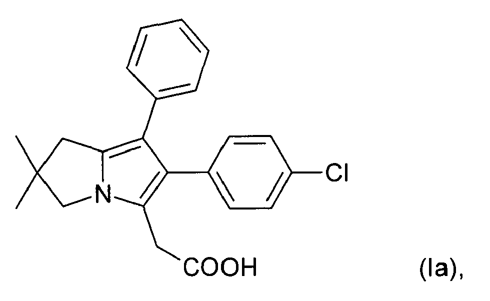 un sel physiologiquement acceptable ou un ester physiologiquement hydrolysable de celui-ci,
(ii) un ou plusieurs agents anti-inflammatoires ulcérogènes destinés à être utilisés en thérapie, et éventuellement
(iii) un support pharmaceutique acceptable,
où l'agent anti-inflammatoire ulcérogène est choisi dans le groupe consistant en l'acide acétylsalicylique (ASA), le salicylate de sodium, l'acétaminophène, la phénacétine, l'ibuprofène, le kétoprofène, l'indométhacine, le flurbiprofène, le diclofénac, le naproxène, le piroxicam, la tébufélone, la nabumétone, le ténidap, l'alcofénac, l'antipyrine, l'aminopyrine, la dipyrone, l'aminopyrone, la phénylbutazone, la clofézone, l'oxyphenbutazone, la prexazone, l'apazone, la benzydamine, le bucolome, le cinchopen, la clonixine, le ditrazol, l'épirizole, le fénoprofène, la floctafénine, l'acide flufénamique, la glaphénine, l'indoprofène, l'acide méclofénamique, l'acide méfénamique, l'acide niflumique, les salidifamides, le sulindac, le suprofène, la tolmétine, la nabumétone, le tiaramide, la proquazone, le bufexamac, le flumizole, la tinoridine, la timégadine, la dapsone, le diflunisal, le bénorylate, le fosfosal, le fenclofénac, le fentiazac, le tilomisole, le carprofène, le fenbufène, l'oxaprozine, l'acide tiaprofénique, le pirprofène, la féprazone, le piroxicam, le sudoxicam, l'isoxicam, le ténoxicam.
